(19) Europäisches Patentamt
European Patent Office
Office européen des brevets

(11) Numéro de publication: **0 226 492 B1**

(12) **FASCICULE DE BREVET EUROPEEN**

(45) Date de publication du fascicule du brevet: **22.03.89**

(51) Int. Cl.⁴: **C 07 C 19/08**, C 07 C 17/20, B 01 J 27/12

(21) Numéro de dépôt: **86402484.9**

(22) Date de dépôt: **06.11.86**

(54) **Procédé de synthèse du chloropentafluoroéthane à partir de dichlorotétrafluoroéthane et d'acide fluorhydrique.**

(30) Priorité: **15.11.85 FR 8516951**

(43) Date de publication de la demande: **24.06.87 Bulletin 87/26**

(45) Mention de la délivrance du brevet: **22.03.89 Bulletin 89/12**

(84) Etats contractants désignés: **AT BE CH DE ES FR GB GR IT LI LU NL SE**

(56) Documents cités:
**DE-A-1 443 184**
**US-A-2 946 828**
**US-A-3 087 974**

(73) Titulaire: **ATOCHEM, 4 & 8, Cours Michelet La Défense 10, F-92800 Puteaux (FR)**

(72) Inventeur: **Azerad, Robert, 1, rue Frédéric Chopin, F-69800 Corbas (FR)**
Inventeur: **Cheminal, Bernard, Lieu-dit La Rivière, F-69530 Orlienas, Brignais (FR)**
Inventeur: **Mathais, Henri, 87, Chemin de l'indiennerie, F-69370 Saint-Didier-au-Mont-d'Or (FR)**

(74) Mandataire: **Leboulenger, Jean et al, ATOCHEM Département Propriété Industrielle, F-92091 Paris la Défense 10 Cédex 42 (FR)**

EP 0 226 492 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne la fabrication du chloropentafluoroéthane $C_2F_5Cl$ par réaction catalytique en phase gazeuse du chlorotétrafluoroéthane $C_2F_4Cl_2$ avec l'acide fluorhydrique HF.

Le chloropentafluoroéthane qui est utilisé comme solvant, agent propulseur ou fluide frigorigène est préparé par des procédés connus par exemple à partir de perchloréthylène, de chlore et d'acide fluorhydrique (brevet de l'Allemagne de l'Est N° 117 580) ou par réaction du trichlorotrifluoroéthane $C_2F_3Cl_3$ en phase gazeuse avec l'acide fluorhydrique en présence de trifluorure d'aluminium (publication japonaise 48-26729/73).

Le brevet US 3087974 décrit la dismutation en phase vapeur de composés chlorofluorés sur un catalyseur sans addition d'acide fluorhydrique. La dismutation du dichlorotétrafluoroéthane se fait selon la réaction:

$$2\ C_2Cl_2F_4 \longrightarrow C_2ClF_5 + C_2Cl_3F_3$$

Ce catalyseur est de l'alumine activée de grande surface qui est traitée par un composé fluorocarboné avant d'être utilisé pour la réaction de dismutation. La conversion du $CF_2ClCF_2Cl$ en $C_2ClF_5$ ne dépasse pas 34% molaire et la production du sous-produit $C_2Cl_3F_3$ est importante.

Le brevet US 3258500 décrit une fluoration catalytique en phase vapeur par l'acide fluorhydrique. Le dichlorotétrafluoroéthane $CF_2Cl-CF_2Cl$ et l'acide fluorhydrique HF dans un rapport molaire

$$\left[\frac{HF}{CF_2Cl-CF_2Cl}\right]$$

compris entre 4 et 5 réagissent à 400° C sur le catalyseur fait d'oxyde de chrome. Le rapport d'hexafluoroéthane $C_2F_6$ formé sur $C_2F_5Cl$ est 0,19 en moles.

Un article de L. MARANGONI et al. «Preparation of chloropentafluoroethane from dichlorotetrafluoroethane» (Journal of Fluorine Chemistry 19, 1981/82, pages 21 à 34) décrit aussi un catalyseur à base d'oxyde de chrome pour la fabrication en phase gazeuse de $C_2F_5Cl$ à partir de $C_2F_4Cl_2$ et HF. La conversion de $C_2Cl_2F_4$ est comprise entre 72 et 75%, le rendement en $C_2F_5Cl$ est compris entre 89 et 92% mais la production d'hexafluoroéthane est encore 8% en moles de la production de $C_2F_5Cl$.

Un article de M. VECCHIO et al. «Studies on a vapor-phase process for the manufacture of chlorofluoroethanes» (Journal of Fluorine Chemistry, 4, 1974, pages 111 à 139) décrit la même réaction que dans l'article précédent mais sur un catalyseur à base de fluorure d'aluminium dopé par des halogénures de nickel, fer et chrome. Le taux de conversion de $C_2F_4Cl_2$ ne dépasse pas 41% et le pourcentage molaire de $C_2F_5Cl$ en sortie de réacteur est 38%.

Les catalyseurs de l'art antérieur sont difficiles à préparer, les rendements et les sélectivités en $C_2F_5Cl$ sont médiocres.

La présente invention surmonte tous ces inconvénients en proposant un procédé simple, souple et économique pour produire le $C_2F_5Cl$.

Le procédé selon l'invention consiste à soumettre le dichlorotétrafluoroéthane $C_2Cl_2F_4$ à l'action de l'acide fluorhydrique HF en phase gazeuse en présence d'un catalyseur préparé en faisant réagir en phase gazeuse une alumine activée dont la teneur en oxyde de sodium est inférieure à 300 ppm et dont le volume des pores de rayon de 40 angströms et plus est supérieur à 0,7 $cm^3/g$ avec de l'acide fluorhydrique ou avec un mélange d'acide fluorhydrique et d'air, d'azote ou d'un composé fluoré.

On obtient une conversion élevée des matières premières supérieure à 80% et un rendement élevé en $C_2F_5Cl$. Un autre avantage du procédé est la durée de vie du catalyseur.

Les alumines activées sont obtenues par chauffage contrôlé d'alumines hydratées de matière à éliminer la plupart de l'eau de constitution (KIRK-OTHMER, Encyclopedia of chemical technology, 3e édition, vol. 2, page 225).

L'alumine utilisée pour préparer le catalyseur est une alumine disponible dans le commerce. Il suffit de choisir une alumine activée dont le volume des pores de 40 Å et plus est supérieur à 0,7 $cm^3/g$ et de préférence compris entre 0,75 et 1 $cm^3/g$. Il est avantageux que l'alumine se présente sous la forme de grains, billes ou extrudés de taille inférieure à une sphère de 20 mm de diamètre et de préférence de quelques millimètres pour permettre une manipulation commode durant le chargement ou la vidange du réacteur.

Comme indiqué précédemment, la teneur en oxyde de sodium $Na_2O$ doit être inférieure à 300 ppm et est, de préférence, la plus faible possible. Il est aussi avantageux de choisir une alumine ne contenant en poids pas plus de 0,5% de silice et pas plus de 0,2% d'oxyde de fer $Fe_2O_3$. Cette alumine est transformée en mélange de trifluorure d'aluminium $AlF_3$ et d'alumine par réaction avec HF seul ou en mélange avec de l'air, de l'azote ou un composé fluoré.

Par exemple, on peut utiliser un mélange de dichlorotétrafluoroéthane $C_2Cl_2F_4$ et d'acide fluorhydrique qu'on fait passer sur cette alumine à une température suffisante pour initier une réaction de transformation de l'alumine et trifluorure d'aluminium. Il est avantageux d'opérer entre 150 et 500° C. On opère de préférence sous la pression atmosphérique avec des temps de contact compris entre 5 et 15 secondes. L'homme de métier peut facilement conduire la réaction et ajuster les proportions de $C_2F_4Cl_2$ et HF, la température, la pression et le temps de contact pour éviter que l'alumine ne soit endommagée par une haute température due à l'exothermicité de la réaction.

Quand il n'y a plus d'évolution de la composition des gaz après passage sur cette alumine, c'est que l'alumine a été tranformée en catalyseur. On peut l'utiliser, éventuellement après un lavage à l'eau, pour faire la fluoration du $C_2F_4Cl_2$ en $C_2F_5Cl$ selon l'invention.

On peut aussi selon un mode de réalisation préféré de l'invention préparer le catalyseur en soumettant l'alumine en lit fluidisé à l'action d'un courant d'air ou d'azote chaud contenant de l'acide fluorhydrique. Il est avantageux d'opérer entre 150 et 500° C. Avantageusement, on utilise un mélange entre 0,1 et 30% molaire d'HF dans l'air ou l'azote et de préférence compris entre 1,5 et 3%. Le débit est avantageusement compris entre 200 et 250 moles/heure par litre de catalyseur. De préférence, on opère à la pression atmosphérique à 350° C ou plus. Il est commode de faire varier la concentration de l'air ou de l'azote en HF pour contrôler l'exothermicité de la réaction. Quand on consomme plus de HF on arrête la réaction et on considère que le catalyseur est prêt pour la fluoration du $C_2F_4Cl_2$.

La réaction entre le dichlorotétrafluoroéthane $C_2F_4Cl_2$ et l'acide fluorhydrique HF anhydre est faite en phase gazeuse sur le catalyseur qui peut être préparé selon l'une des deux voires précédentes. La température est avantageusement comprise entre 350 et 550° C et de préférence entre 380 et 500° C. Le rapport molaire $HF/C_2Cl_2F_4$ est avantageusement compris entre 0,5 et 1,5 et de préférence entre 0,9 et 1,1. Bien qu'on puisse opérer à toute pression, pourvu qu'on reste en phase gazeuse, il est cependant beaucoup plus simple d'opérer entre 0,5 et 4 bar absolus et de préférence à la pression atmosphérique ou à une pression voisine, et avec un temps de contact compris entre 5 et 15 secondes et de préférence compris entre 6 et 13 secondes.

Les exemples suivants illustrent l'invention sans la limiter.

Dans tous ces exemples le $C_2F_4Cl_2$ utilisé contient 92,7% d'isomère symétrique.

*Exemple 1*

On utilise une alumine pure activée fournie par la Société KAISER (réf. A1 4192) sous forme d'extrudés de 0,8 mm (1/32") dont les caractéristiques sont les suivantes:

Volume des pores de rayon supérieur ou égal à 40Å = 0,91 $cm^3/g$

Surface spécifique totale BET = 161 $m^2/g$

Rayon moyen des pores = 106 Å

Densité apparente (tassé en vrac) = 0,47

Surface des pores d'un rayon compris entre 50 et 250 Å = 105 $m^2/g$

Teneur en $Fe_2O_3$: 0,08% en poids

Teneur en $SiO_2$: 0,13% en poids

Teneur en $Na_2O$: 0,015% en poids

On charge 0,12 litre de cette alumine en lit fixe dans un réacteur tubulaire de diamètre intérieur 28 mm puis on la transforme en catalyseur en opérant comme dans le tableau ci-dessous.

| Température dans le réacteur (° C) | Pression absolue (atmosphères) | Débit molaire de réactifs en moles/(h × 100 ml de catalyseur) | | Temps de contact (secondes) | Durée (heures cumulées) |
|---|---|---|---|---|---|
| | | $C_2Cl_2F_4$ | HF | | |
| 350 | 1 | 0,374 | 0,177 | 12,8 | 48 |
| 380 | 1 | 0,328 | 0,178 | 12,7 | 93 |
| 410 | 1 | 0,337 | 0,187 | 12,2 | 142 |

A l'issue de ce traitement, après lavage des hydracides à l'eau et à l'aide d'une solution aqueuse de soude, le taux de $C_2ClF_5$ dans l'effluent gazeux du réacteur atteint 23,6%, alors qu'il n'était que de 0,7% à 350° C.

La température du réacteur est alors portée à 450° C, le $C_2Cl_2F_4$ étant de même qualité que précédemment. Les résultats sont reportés dans le tableau I suivant, où les débits molaires sont exprimés pour 0,1 litre de catalyseur, la pression du réacteur étant 1 bar absolu, et la température maintenue à 450° C. La durée de fonctionnement du catalyseur qui est indiquée comprend l'étape précédente de transformation de l'alumine en catalyseur.

Dans tous les essais précédemment décrits le dichlorotétrafluoroéthane non transformé (environ 15% en moles du composé $C_2Cl_2F_4$ engagé) contient 20 à 25% d'isomère symétrique $CF_2Cl-CF_2Cl$.

Après 550 heures de fonctionnement dans les conditions opératoires décrites plus haut et une opération à 450° C avec de l'air pur, le catalyseur fournit des résultats absolument identiques aux précédents.

*Exemple 2*

On prend la même alumine activée que dans l'exemple 1 sous la même forme physique, des extrudés de 0,8 mm, et on la transforme en catalyseur par une technique en lit fluidisé. On prend 0,125 litre d'alumine KAISER 4192, soit 51,3 g, on la chauffe à 350° C dans un courant d'azote puis on introduit pendant 24 h le mélange gazeux suivant (air: 27,2 moles/h; HF: 0,77 mole/h) à pression atmosphérique.

Au bout de 24 heures de ce traitement, le catalyseur contient 62% de fluor, c'est-à-dire 91,4% de fluorure d'aluminium et 8,6% d'alumine non transformée. Son poids atteint 77,9 grammes.

On prélève 68,1 grammes de ce catalyseur (0,1 litre) pour le tester dans le même réacteur tubulaire de 28 mm de diamètre que dans l'exemple 1, et on opère comme dans l'exemple 1. Les résultats sont reportés dans le tableau II ci-après.

La durée de fonctionnement du catalyseur qui

est indiquée dans ce tableau correspond à la durée depuis le changement du réacteur en lit fixe et ne comprend pas la transformation de l'alumine en catalyseur.

*Exemple 3*

On opère comme dans l'exemple 2, à partir de la même alumine activée sauf qu'après la transformation de l'alumine en catalyseur et chargement du réacteur en lit fixe de fluoration de $C_2F_4Cl_2$, on démarre la réaction avec un rapport molaire $HF/C_2Cl_2F_4$ voisin de 1 au lieu de 0,4 dans l'exemple 2.

Les résultats sont reportés sur le tableau III.

Le mode de démarrage ne modifie pas l'activité ni la sélectivité du catalyseur.

La durée de fonctionnement qui est indiquée est la durée depuis le chargement du réacteur en lit fixe et ne comprend pas la transformation de l'alumine en catalyseur.

*Exemple 4* (comparatif)

On opère comme dans l'exemple 2, mais en utilisant une alumine activée dont le volume poreux est différent de l'alumine activée utilisée dans le procédé selon l'invention, on utilise l'alumine SCM 250 ayant les caractéristiques suivantes:

Pureté chimique
- $Na_2O$: 800 ppm
- $Fe_2O_3$: 300 ppm
- $SiO_2$: 200 ppm

Propriétés physiques
- Forme: billes de 2 à 4 mm de diamètre
- Surface spécifique (BET) = 270 $m^2/g$
- Volume total des pores de rayon supérieur ou égal à 40 Å = 0,63 $cm^3/g$
- Diamètre moyen de pores 90 Å
- Densité en vrac = 0,66 g/ml

On obtient un catalyseur dont la teneur en $AlF_3$ est 87,7% en poids, le reste étant de l'alumine non transformée. Les résultats sont reportés dans le tableau IV,1, les durées de fonctionnement sont mesurées comme dans l'exemple 2.

On utilise la même alumine SCM 250 en sphères de 2 à 4 mm et on la transforme en catalyseur comme précédemment, mais après la transformation on la réduit mécaniquement en petits morceaux inférieurs à 1 mm pour se rapprocher des dimensions de l'alumine KAISER des exemples 1, 2 et 3. On utilise alors ce catalyseur comme précédemment.

Les résultats sont reportés dans le tableau IV,2.

Les durées de fonctionnement figurant dans le tableau ne comprennent pas le temps de transformation de l'alumine en catalyseur.

*Exemple 5*

On utilise l'alumine CS 331-1 vendue par la société CATALYSTS AND CHEMICALS EUROPE (CCE) dont les principales caractéristiques sont les suivantes:
- forme: extrudés de 1/16" (≃ 1,5 mm) de diamètre
- surface active (BET): 225 $m^2/g$
- rayon moyen des pores: 90 Å
- volume des pores de rayon supérieur ou égal à 40 Å: 0,76 $cm^3/g$
- densité en vrac: 0,60 g/ml
- teneur en $Na_2O$: 0,02%
- teneur en $Fe_2O_3$: 0,08%
- teneur en $SiO_2$: 0,03%

Le catalyseur est préparé comme dans l'exemple 2, la concentration en $AlF_3$ du catalyseur est 92%, avec 8% d'alumine non transformée.

Les résultats des essais correspondants figurent dans le tableau V ci-après; on constate que les résultats obtenus sont nettement supérieurs à ceux obtenus dans l'exemple comparatif N° 4 (Alumine SCM 250).

Les durées de fonctionnement indiquées ne comprennent pas la transformation de l'alumine en catalyseur.

*Exemple 6* (comparatif)

Fluoration de $C_2F_4Cl_2$ par HF selon un procédé non conforme à l'invention.

Au lieu de prendre une alumine et de la transformenr en catalyseur comme précédemment, on utilise du trifluorure d'aluminium commercial en poudre contenant environ 8% d'alumine.

Ses principales caractéristiques physico-chimiques sont les suivantes:
- granulométrie moyenne de la poudre: 50 à 80 microns
- aire spécifique totale (BET) = 1,6 $m^2/g$
- volume des pores de rayon supérieur ou égal à 40 Å: 0,25 $cm^3/g$

Puis on fait, comme dans les exemples précédents, la fluoration du $C_2F_4Cl_2$ par HF mais en lit fluide au lieu d'un lit fixe.

Le tableau VI rassemble les résultats.

*Exemple 7*

On opère comme dans l'exemple 2, sur le même type d'alumine (KAISER 4192), mais on travaille avec un temps de contact plus faible.

Les résultats sont reportés dans le tableau VII où les durées de fonctionnement ne comprennent pas le temps de transformation de l'alumine en catalyseur.

On constate que la productivité en $C_2ClF_5$ peut être augmentée de manière importante sans nuire au rendement.

Le tableau VIII, qui rassemble des résultats obtenus dans les conditions opératoires analogues, montre l'influence de la teneur en $Na_2O$.

*Tableau I*

| CONDITIONS OPÉRATOIRES | | | | | RÉSULTATS OBTENUS | | | | | | |
|---|---|---|---|---|---|---|---|---|---|---|---|
| Durée de fonctionnement du catalyseur (heures cumulées) | Débit molaire de réactifs (moles/heure) | | Rapport molaire $HF/C_2Cl_2F_4$ | Temps de contact (secondes) | Taux de conversion (%) | | Taux de transformation du $C_2Cl_2F_4$ en $C_2ClF_5$ (%) | Rapport molaire $\frac{C_2F_6}{C_2ClF_5}$ (× 100) | Rapport molaire $\frac{C_2F_3Cl_3}{C_2ClF_5}$ (× 100) | Rapport molaire $\frac{CF_4+CF_3Cl}{C_2ClF_5}$ (× 100) | Productivité en grammes de $C_2ClF_5$ par heure par litre de calalyseur |
| | HF | $C_2Cl_2F_4$ | | | HF | $C_2Cl_2F_4$ | | | | | |
| 165 | 0,132 | 0,330 | 0,40 | 13,1 | 90,0 | 76,9 | 62,0 | 0,5 | 20,1 | 0,2 | 316 |
| 257 | 0,268 | 0,257 | 1,04 | 11,6 | 79,3 | 85,2 | 81,4 | 0,6 | 3,9 | 0,1 | 310 |
| 445 | 0,254 | 0,283 | 0,90 | 11,3 | 73,1 | 85,5 | 81,7 | 0,5 | 4,0 | 0,1 | 351 |

*Tableau II*

| CONDITIONS OPÉRATOIRES | | | | RÉSULTATS OBTENUS | | | | | |
|---|---|---|---|---|---|---|---|---|---|
| Durée de fonctionnement du catalyseur (heures cumulées) | Débit molaire de réactifs (moles/heure) | | Temps de contact (secondes) | Taux de conversion (%) | | Taux de transformation du $C_2Cl_2F_4$ en $C_2ClF_5$ (%) | Rapport molaire $\frac{C_2F_6+CF_4}{C_2ClF_5}$ (× 100) | Rapport molaire $\frac{C_2F_3Cl_3}{C_2ClF_5}$ (× 100) | Productivité en grammes de $C_2ClF_5$ par heure par litre de calalyseur |
| | HF | $C_2Cl_2F_4$ | | HF | $C_2Cl_2F_4$ | | | | |
| 23,5 | 0,140 | 0,377 | 11,8 | 89,3 | 80,4 | 64,7 | 1,1 | 19,2 | 377 |
| 46,5 | 0,149 | 0,365 | 11,8 | 91,1 | 80,5 | 65,1 | 1,2 | 18,8 | 367 |
| 66,5 | 0,140 | 0,360 | 12,2 | 93,4 | 79,6 | 63,8 | 0,7 | 20,0 | 354 |
| 89,5 | 0,244 | 0,247 | 12,4 | 79,5 | 87,9 | 80,8 | 1,0 | 5,5 | 309 |
| 111,5 | 0,223 | 0,247 | 12,9 | 80,2 | 87,2 | 79,6 | 1,0 | 6,0 | 304 |
| 133,5 | 0,218 | 0,247 | 13,0 | 83,1 | 85,8 | 77,2 | 1,0 | 7,5 | 295 |

*Conditions opératoires*

- Catalyseur en lit fixe (volume = 0,1 litre)
- Température: 450° C
- Pression: atmosphérique

*Tableau III*

| CONDITIONS OPÉRATOIRES | | | | | RÉSULTATS OBTENUS | | | | | |
|---|---|---|---|---|---|---|---|---|---|---|
| Durée de fonctionnement du catalyseur (heures cumulées) | Débit molaire de réactifs (moles/heure) | | Rapport molaire $HF/C_2Cl_2F_4$ | Temps de contact (secondes) | Taux de conversion (%) | | Taux de transformation du $C_2Cl_2F_4$ en $C_2ClF_5$ (%) | Rapport molaire $\frac{C_2F_6+CF_4}{C_2ClF_5}$ (× 100) | Rapport molaire $\frac{C_2Cl_3F_3}{C_2ClF_3}$ (× 100) | Productivité en grammes de $C_2ClF_5$ par heure par litre de calalyseur |
| | HF | $C_2Cl_2F_4$ | | | HF | $C_2Cl_2F_4$ | | | | |
| 24 | 0,255 | 0,243 | 1,05 | 13,2 | 75,4 | 86,5 | 79,8 | 1,2 | 4,5 | 300 |
| 47 | 0,240 | 0,245 | 0,98 | 12,5 | 76,8 | 86,7 | 79,9 | 0,9 | 5,2 | 302 |

*Conditions opératoires*

- Catalyseur en lit fixe (volume = 0,1 litre) préparé comme dans l'exemple n° 2
- Température = 450° C
- Pression atmosphérique

*Tableau IV,1*

| CONDITIONS OPÉRATOIRES | | | | | RÉSULTATS OBTENUS | | | | | |
|---|---|---|---|---|---|---|---|---|---|---|
| Durée de fonctionnement du catalyseur (heures cumulées) | Débit molaire de réactifs (moles/heure) | | Rapport molaire $HF/C_2Cl_2F_4$ | Temps de contact (secondes) | Taux de conversion (%) | | Taux de transformation du $C_2Cl_2F_4$ en $C_2ClF_5$ (%) | Rapport molaire $\frac{C_2F_6+CF_4}{C_2ClF_5}$ (× 100) | Rapport molaire $\frac{C_2Cl_3F_3}{C_2ClF_5}$ (× 100) | Productivité en grammes de $C_2ClF_5$ par heure par litre de calalyseur |
| | HF | $C_2Cl_2F_4$ | | | HF | $C_2Cl_2F_4$ | | | | |
| 24 | 0,240 | 0,243 | 0,99 | 12,5 | 9,0 | 9,4 | 6,8 | 5,8 | 4,3 | 25 |
| 48 | 0,235 | 0,253 | 0,93 | 12,4 | 10,0 | 10,3 | 8,2 | 2,4 | 3,6 | 32 |
| 71 | 0,249 | 0,253 | 0,98 | 12,1 | 12,7 | 10,3 | 7,9 | 1,9 | 3,7 | 31 |

*Conditions opératoires communes aux essais*

- Catalyseur en lit fixe (volume = 0,1 litre) en billes de 2 à 4 mm
- Température = 450° C
- Pression atmosphérique

*Tableau IV,2*

| CONDITIONS OPÉRATOIRES | | | | | RÉSULTATS OBTENUS | | | | | |
|---|---|---|---|---|---|---|---|---|---|---|
| Durée de fonctionnement du catalyseur (heures cumulées) | Débit molaire de réactifs (moles/heure) | | Rapport molaire $HF/C_2Cl_2F_4$ | Temps de contact (secondes) | Taux de conversion % | | Taux de transformation du $C_2Cl_2F_4$ en $C_2ClF_5$ (%) | Rapport molaire $\frac{C_2F_6+CF_4}{C_2ClF_5}$ (× 100) | Rapport molaire $\frac{C_2Cl_3F_3}{C_2ClF_5}$ (× 100) | Productivité en grammes de $C_2ClF_5$ par heure par litre de catalyseur |
| | HF | $C_2Cl_2F_4$ | | | HF | $C_2Cl_2F_4$ | | | | |
| 117 | 0,232 | 0,233 | 1,0 | 13,0 | 9,5 | 10,6 | 8,4 | 0,6 | 2,3 | 30 |
| 139 | 0,239 | 0,234 | 1,0 | 12,8 | 8,9 | 10,3 | 8,0 | 0,6 | 2,4 | 29 |
| Température = 500° C après la 139e heure | | | | | | | | | | |
| 159 | 0,238 | 0,230 | 1,0 | 12,1 | 10,9 | 20,3 | 17,2 | 1,5 | 1,1 | 61 |

*Conditions opératoires*

- Catalyseur en lit fixe (volume = 0,1 litre)
- Température = 450° C jusqu'à la 139e heure, puis 500° C
- Pression atmosphérique

*Tableau V*

| CONDITIONS OPÉRATOIRES | | | | | RÉSULTATS OBTENUS | | | | | |
|---|---|---|---|---|---|---|---|---|---|---|
| Durée de fonctionnement du catalyseur (heures cumulées) | Débit molaire de réactifs (moles/heure) | | Rapport molaire $HF/C_2Cl_2F_4$ | Temps de contact (secondes) | Taux de conversion (%) | | Taux de transformation du $C_2Cl_2F_4$ en $C_2ClF_5$ (%) | Rapport molaire $\frac{C_2F_6+CF_4}{C_2ClF_5}$ (× 100) | Rapport molaire $\frac{C_2Cl_3F_3}{C_2ClF_5}$ (× 100) | Productivité en grammes de $C_2ClF_5$ par heure par litre de catalyseur |
| | HF | $C_2Cl_2F_4$ | | | HF | $C_2Cl_2F_4$ | | | | |
| 25 | 0,245 | 0,242 | 1,0 | 12,5 | 67,5 | 76,9 | 71,5 | 1,7 | 5,3 | 268 |
| 48 | 0,245 | 0,245 | 1,0 | 12,4 | 66,4 | 77,0 | 71,0 | 1,0 | 5,4 | 268 |
| 71 | 0,245 | 0,245 | 1,0 | 12,4 | 66,8 | 76,9 | 70,7 | 1,0 | 5,7 | 268 |
| 86,5 | 0,194 | 0,243 | 0,8 | 13,9 | 66,2 | 78,3 | 70,8 | 1,2 | 7,5 | 266 |

*Conditions opératoires*

- Catalyseur en lit fixe (volume = 0,1 litre) en extrudés de 1,6 mm, préparé comme dans l'exemple N° 2
- Température = 450° C
- Pression atmosphérique

*Tableau VI*

| CONDITIONS OPÉRATOIRES | | | | | RÉSULTATS OBTENUS | | | | | |
|---|---|---|---|---|---|---|---|---|---|---|
| Durée de fonctionnement du catalyseur (heures cumulées) | Débit molaire de réactifs (moles/heure) | | Rapport molaire $HF/C_2Cl_2F_4$ | Temps de contact (secondes) | Taux de conversion (%) | | Taux de transformation du $C_2Cl_2F_4$ en $C_2ClF_5$ (%) | Rapport molaire $\frac{C_2F_6+CF_4}{C_2ClF_5}$ (× 100) | Rapport molaire $\frac{C_2Cl_3F_3}{C_2ClF_5}$ (× 100) | Productivité en grammes de $C_2ClF_5$ par heure par litre de calalyseur |
| | HF | $C_2Cl_2F_4$ | | | HF | $C_2Cl_2F_4$ | | | | |
| 24 | 0,357 | 0,358 | 1,0 | 11,8 | 7,0 | 0,2 | 0,1 | 0 | 0 | 0,4 |
| 47 | 0,351 | 0,378 | 0,9 | 11,6 | 2,0 | 0,1 | 0,1 | 0 | 0 | 0,4 |
| 70 | 0,351 | 0,376 | 0,9 | 11,6 | 4,1 | 0,1 | 0,1 | 0 | 0 | 0,4 |
| 92 | 0,360 | 0,368 | 1,0 | 10,8 | 5,4 | 0,2 | 0,1 | 0 | 0 | 0,4 |

*Conditions opératoires*

- Catalyseur en lit fluide (volume = 0,14 litre) de granulométrie moyenne 50 à 80 microns
- Température = 450° C
- Pression atmosphérique

*Tableau VII*

| CONDITIONS OPÉRATOIRES | | | | | RÉSULTATS OBTENUS | | | | | |
|---|---|---|---|---|---|---|---|---|---|---|
| Durée de fonctionnement du catalyseur (heures cumulées) | Débit molaire de réactifs (moles/heure) | | Rapport molaire $HF/C_2Cl_2F_4$ | Temps de contact (secondes) | Taux de conversion (%) | | Taux de transformation du $C_2Cl_2F_4$ en $C_2ClF_5$ (%) | Rapport molaire $\frac{C_2F_6+CF_4}{C_2ClF_5}$ (× 100) | Rapport molaire $\frac{C_2Cl_3F_3}{C_2Cl_3F_3}$ (× 100) | Productivité en grammes de $C_2ClF_5$ par heure par litre de calalyseur |
| | HF | $C_2Cl_2F_4$ | | | HF | $C_2Cl_2F_4$ | | | | |
| 24 | 0,349 | 0,375 | 0,93 | 8,4 | 60,3 | 65,4 | 60,2 | 1,0 | 7,5 | 348,8 |
| 48 | 0,367 | 0,373 | 0,98 | 8,2 | 59,5 | 67,2 | 62,5 | 0,5 | 7,0 | 360,2 |
| 72,45 | 0,521 | 0,501 | 1,04 | 5,9 | 51,7 | 60,9 | 57,1 | 0,5 | 6,1 | 442,0 |
| 95,45 | 0,500 | 0,495 | 1,01 | 6,4 | 54,4 | 62,7 | 58,5 | 0,7 | 6,7 | 447,4 |

*Conditions opératoires*

- Catalyseurs en lit fixe (volume = 0,1 litre) en extrudés de 0,8 mm
- Température = 450° C
- Pression atmosphérique

*Tableau VIII*

| CONDITIONS OPÉRATOIRES | | | | | RÉSULTATS OBTENUS | | | | |
|---|---|---|---|---|---|---|---|---|---|
| Teneur en $Na_2O$ (ppm) | Débit molaire de réactifs (moles/heure) | | Rapport molaire $HF/C_2Cl_2F_4$ | Temps de contact (secondes) | Taux de conversion (%) | | Taux de transformation du $C_2Cl_2F_4$ en $C_2ClF_5$ (%) | Rapport molaire $\frac{C_2Cl_3F_3}{C_2ClF_5}$ (× 100) | Productivité en grammes de $C_2ClF_5$ par heure par litre de catalyseur |
| | HF | $C_2Cl_2F_4$ | | | HF | $C_2Cl_2F_4$ | | | |
| 150 | 0,449 | 0,429 | 1,05 | 7,4 | 52,1 | 61,1 | 57,9 | 5,5 | 383,8 |
| 930 | 0,411 | 0,428 | 0,96 | 7,2 | 7,8 | 8,1 | 7,7 | 5,2 | 50,9 |

*Conditions opératoires*

- Catalyseurs en lit fixe (volume = 0,1 litre) en extrudés de 0,8 mm
- Température = 450° C
- Pression atmosphérique

## Revendications

1. Procédé de fabrication en phase gazeuse du chloropentafluoroéthane par action de l'acide fluorhydrique sur le dichlorotétrafluoroéthane en présence d'un catalyseur, caractérisé en ce que le catalyseur est préparé en faisant réagir en phase gazeuse une alumine activée dont la teneur en oxyde de sodium est inférieure à 300 ppm et dont le volume des pores de rayon de 40 angströms et plus est supérieur à 0,7 $cm^3/g$ avec de l'acide fluorhydrique ou avec un mélange d'acide fluorhydrique et d'air, d'azote ou d'un composé fluoré.

2. Procédé selon la revendication 1, caractérisé en ce qu'on utilise une alumine dont le volume des pores de rayon de 40 Å et plus est compris entre 0,75 et 1 $cm^3/g$.

3. Procédé selon la revendication 1 ou 2, caractérisé en ce que l'alumine a une pureté supérieure à 99,2% en poids.

4. Procédé selon l'une des revendications 1 à 3, caractérisé en ce que l'alumine est sous forme de particules de taille inférieure à une sphère de 20 mm de diamètre.

5. Procédé selon l'une des revendications 1 à 4, caractérisé en ce que la préparation du catalyseur est faite par action d'un mélange d'acide fluorhydrique et d'air ou d'azote sur l'alumine à une température comprise entre 150 et 500° C.

6. Procédé selon la revendication 5, caractérisé en ce que la teneur de HF dans le mélange HF-air ou azote est comprise entre 0,1 et 30% et, de préférence, entre 1,5 et 3% en moles.

7. Procédé selon la revendication 5 ou 6, dans lequel on opère à la pression atmosphérique à 350° C ou plus.

8. Procédé selon l'une des revendications 1 à 4, caractérisé en ce que la préparation du catalyseur est faite par action en phase gazeuse d'un mélange d'acide fluorhydrique et de dichlorotétrafluoroéthane sur l'alumine à une température comprise entre 150 et 500° C.

9. Procédé selon la revendication 8, caractérisé en ce qu'on opère à la pression atmosphérique avec des temps de contact compris entre 5 et 15 secondes.

10. Procédé selon l'une des revendications 1 à 9, caractérisé en ce que la fluoration du dichlorotétrafluoroéthane par HF est faite avec un rapport molaire HF/dichlorotétrafluoroéthane compris entre 0,5 et 1,5 et, de préférence, compris entre 0,9 et 1,1, et à une température comprise entre 350 et 550° C et, de préférence, entre 380 et 500° C.

11. Procédé selon la revendication 10, caractérisé en ce qu'on opère entre 0,5 et 4 bar absolus.

12. Procédé selon la revendication 11, caractérisé en ce qu'on opère à la pression atmosphérique avec des temps de contact compris entre 5 et 15 secondes et, de préférence, entre 6 et 13 secondes.

## Patentansprüche

1. Verfahren zur Herstellung von Chlorpentafluorethan in der Gasphase durch Einwirkung von Fluorwasserstoffsäure auf Dichlortetrafluorethan in Gegenwart eines Katalysators, dadurch gekennzeichnet, dass der Katalysator durch Reagierenlassen eines aktiven Aluminiumoxids in der Gasphase, dessen Gehalt an Natriumoxid geringer als 300 ppm ist und dessen Volumen der Poren mit einem Radius von 40 Angström oder mehr grösser ist als 0,7 $cm^3/g$, mit Fluorwasserstoffsäure oder mit einem Gemisch von Fluorwasserstoffsäure und Luft, Stickstoff oder einer fluorierten Verbindung hergestellt wird.

2. Verfahren nach Anspruch 1, dadurch gekennzeichnet, dass man ein Aluminiumoxid verwendet, dessen Volumen der Poren mit einem Radius von 40 Angström und mehr zwischen 0,75 und 1 $cm^3/g$ beträgt.

3. Verfahren nach Anspruch 1 oder 2, dadurch gekennzeichnet, dass das Aluminiumoxid eine Reinheit von mehr als 99,2 Gew.% hat.

4. Verfahren nach einem der Ansprüche 1 bis 3, dadurch gekennzeichnet, dass das Aluminiumoxid in Form von Partikeln einer Grösse kleiner als eine Kugel mit 20 mm Durchmesser vorliegt.

5. Verfahren nach einem der Ansprüche 1 bis 4, dadurch gekennzeichnet, dass die Herstellung des Katalysators durch Einwirkung eines Gemischs aus Fluorwasserstoffsäure und Luft oder Stickstoff auf Aluminiumoxid bei einer Temperatur zwischen 150 und 500° C erfolgt.

6. Verfahren nach Anspruch 5, dadurch gekennzeichnet, dass der Gehalt an HF in dem Gemisch HF-Luft oder -Stickstoff zwischen 0,1 und 30% und vorzugsweise zwischen 1,5 und 3 Mol.% liegt.

7. Verfahren nach Anspruch 5 oder 6, bei dem man bei atmosphärischem Druck bei einer Temperatur von 350° C oder mehr arbeitet.

8. Verfahren nach einem der Ansprüche 1 bis 4, dadurch gekennzeichnet, dass die Herstellung des Katalysators durch Einwirkung eines Gemischs von Fluorwasserstoffsäure und Dichlortetrafluorethan in der Gasphase auf Aluminiumoxid bei einer Temperatur zwischen 150 und 500° C geschieht.

9. Verfahren nach Anspruch 8, dadurch gekennzeichnet, dass man bei atmosphärischem Druck arbeitet mit einer Kontaktzeit von 5 bis 15 Sekunden.

10. Verfahren nach einem der Ansprüche 1 bis 9, dadurch gekennzeichnet, dass die Fluorierung des Dichlortetrafluorethans mit HF mit einem molaren Verhältnis HF/Dichlortetrafluorethan zwischen 0,5 und 1,5 und vorzugsweise zwischen 0,9 und 1,1 und bei einer Temperatur zwischen 350 und 550° C und vorzugsweise zwischen 380 und 500° C durchgeführt wird.

11. Verfahren nach Anspruch 10, dadurch gekennzeichnet, dass man zwischen 0,5 und 4 bar absolut arbeitet.

12. Verfahren nach Anspruch 11, dadurch gekennzeichnet, dass man bei atmosphärischem Druck mit einer Kontaktzeit von 5 bis 15 Sekunden und vorzugsweise zwischen 6 und 13 Sekunden arbeitet.

## Claims

1. Gas phase process for the manufacture of chloropentafluoroethane by the action of hydrofluoric acid on dichlorotetrafluoroethane in the presence of a catalyst, characterized in that the catalyst is prepared by reacting, in a gaseous phase, an activated alumina whose sodium oxide content is less than 300 ppm and in which the volume of the pores with a radius of 40 angstroms and more is greater than 0.7 $cm^3/g$ with hydrofluoric acid or with a mixture of hydrofluoric acid with air, nitrogen or a fluoro compound.

2. Process according to Claim 1, characterized in that an alumina is used in which the volume of the pores with a radius of 40 A and more is between 0.75 and 1 $cm^3/g$.

3. Process according to Claim 1 or 2, characterized in that the alumina has a purity greater than 99.2% by weight.

4. Process according to one of Claims 1 to 3, characterized in that the alumina is in the form of particles of a size smaller than a sphere with a diameter of 20 mm.

5. Process according to one of Claims 1 to 4, characterized in that the preparation of the catalyst is carried out by the action of a mixture of hydrofluoric acid and air or nitrogen on alumina at a temperature of between 150 and 500° C.

6. Process according to Claim 5, characterized in that the HF content in the mixture of HF and air or nitrogen is between 0.1 and 30% and preferably between 1.5 and 3% on a molar basis.

7. Process according to Claim 5 or 6, in which the operation is carried out at atmospheric pressure at 350° C or above.

8. Process according to one of Claims 1 to 4, characterized in that the preparation of the catalyst is carried out by a gaseous phase reaction of a mixture of hydrofluoric acid and dichlorotetrafluoroethane over alumina at a temperature of between 150 and 500° C.

9. Process according to Claim 8, characterized in that the operation is carried out at atmospheric pressure with contact times of between 5 and 15 seconds.

10. Process according to one of Claims 1 to 9, characterized in that the fluorination of dichlorotetrafluoroethane with HF is carried out with a molar ratio HF/dichlorotetrafluoroethane of between 0.5 and 1.5 and preferably between 0.9 and 1.1, and at a temperature of between 350 and 550° C and preferably between 380 and 500° C.

11. Process according to Claim 10, characterized in that the operation is carried out between 0.5 and 4 bar absolute.

12. Process according to Claim 11, characterized in that the operation is carried out at atmospheric pressure with contact times of between 5 and 15 seconds and preferably between 6 and 13 seconds.